(19) Europäisches Patentamt
European Patent Office
Office européen des brevets

(11) Veröffentlichungsnummer: **0 338 428 B1**

(12) **EUROPÄISCHE PATENTSCHRIFT**

(45) Veröffentlichungstag der Patentschrift: **23.06.93**

(51) Int. Cl.$^5$: **C09C 1/00**

(21) Anmeldenummer: **89106600.3**

(22) Anmeldetag: **13.04.89**

(54) **Metalloxidbeschichtete Aluminiumpigmente.**

(30) Priorität: **21.04.88 DE 3813335**

(43) Veröffentlichungstag der Anmeldung:
**25.10.89 Patentblatt 89/43**

(45) Bekanntmachung des Hinweises auf die Patenterteilung:
**23.06.93 Patentblatt 93/25**

(84) Benannte Vertragsstaaten:
**BE CH DE ES FR GB IT LI NL SE**

(56) Entgegenhaltungen:
**EP-A- 0 033 457**
**EP-A- 0 106 235**

**CHEMICAL ABSTRACTS, Band 101, Nr. 22, 26. November 1984, Seite 92, Zusammenfassung Nr. 193781r, Columbus, Ohio, US; & JP-A-59 126 468**

(73) Patentinhaber: **BASF Aktiengesellschaft**
**Carl-Bosch-Strasse 38**
**W-6700 Ludwigshafen(DE)**

(72) Erfinder: **Ostertag, Werner, Dr.**
**Oberer Bergelweg 2**
**W-6718 Gruenstadt(DE)**
Erfinder: **Mronga, Norbert, Dr.**
**Ringstrasse 2**
**W-6915 Dossenheim(DE)**

EP 0 338 428 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung bezieht sich auf mit Titanoxiden belegte Aluminium-Pigmente, ihre Herstellung und Verwendung.

Diese metallisch glänzenden Reflexionspigmente zählen zu der Gruppe der Effekt-Pigmente. Effektpigmente sind plättchenförmige Pigmente, deren optischer Eindruck, wenn sie ausgerichtet appliziert werden, winkelabhängig ist. Effektpigmente finden in hochwertigen Lacken, z.B. Automobillacken, Kunststoffen, in der dekorativen Kosmetik, im Druck und in der Keramik Anwendung.

Im speziellen handelt es sich bei den erfindungsgemäßen Pigmenten um metallische Interferenzreflexionspigmente, d.h. um Pigmente, die aus einem Substrat, das Spiegelreflexion zeigt, und aus einer Beschichtung, die Interferenzfarben aufweist, bestehen.

Die Gruppe derzeit bekannter metallischer Interferenzreflexionspigmente ist klein.

Zu ihr gehören die al-bekannten Kupfert und Messingpigmente, welche durch kontrollierte Oxidation ihrer Oberfläche auf ein bestimmte Anlauffarbe eingestellt sind. Der auf der Oberfläche solcher Kupfer- und Messingpigmente sich bildende interferenzfähige Belag besteht stofflich stets aus den Oxiden des jeweiligen metallischen Substrats.

In EP-A-33 457 sind metallische Interferenzreflexionspigmente beschrieben, die aus einem $Fe_2O_3$-beschichteten Al-Substrat bestehen. In diesem Fall besteht der interferenzfähige Belag nicht aus dem Oxid des metallischen Substrats. Die Pigmente werden durch kontrollierte Verbrennung von Eisenpentacarbonyl in einem Wirbelbett aus erwärmten Al-Plättchen hergestellt und zeigen je nach Dicke der $Fe_2O_3$-Beschichtung gelbe, orange, rote bis violette Interferenzfarben.

In allen Fällen derzeit bekannter metallischer Interferenzreflexionspigmente ist der oxidische Oberflächenbelag bunt. Dies bedeutet jedoch, daß der Farbeindruck solcher Pigmente nicht allein auf Interferenz, sondern auch auf Absorption zurückzuführen ist. Stets beruht er auf einem Zusammenwirken von Absorptions- und Interferenzfarbe.

Für die Farbtonbrillanz kann dies von Vorteil, aber auch von Nachteil sein. Höchste Farbtonbrillanz wird bei solchen Pigmenten beobachtet, deren schichtdickenabhängige Interferenzfarbe nahe an der Absorptionsfarbe der oxidischen Beschichtung liegt. Beispielsweise sind rote $Fe_2O_3$-belegte Al-Pigmente (rote Interferenzfarbe + rote Absorptionsfarbe)sehr fartonbrillant. Geringe Farbtonbrillanz weisen Pigmente auf, deren Interferenzfarbe weit entfernt von der Absorptionsfarbe liegt. Beispielsweise sind $Fe_2O_3$-belegte Al-Pigmente, welche schichtdickenanbhängig eine blaue Interferenzfarbe haben, braungrau und stumpf. In diesem Falle führt das Zusammenwirken von blauer Interferenzfarbe und roter Absorptionsfarbe zu einer Verminderungder Farbtonbrillanz.

Da alle bisher bekannten metallischen Interferenzreflexionspigmente aufgrund der Buntheit des oxidischen Belags einen Bereich aufweisen, in dem ihre Farbtonbrillanz beeinträchtigt ist, versteht es sich von selbst, daß es für den Pigmentfachmann von Interesse war, metallische Interferenzreflexionspigmente zu entwickeln, deren Beschichtungsdicke variiert werden kann, ohne daß die Farbtonbrillanz der jeweiligen Interferenzfarbe durch die Absorptionsfarbe der Beschichtung beeinträchtigt wird.

EP-A-328906 betrifft Aluminium pigmente, die durch wäßrige Hydrolyse von Titantetraisopropanolgt in Isopropanol mit Titanoxid beschichtet wurden.

Aufgabe der vorliegenden Erfindung war somit die Entwicklung metallischer Interferenzreflexionspigmente, bei denen ein metallisches Substrat mit einer nicht absorbierenden interferenzfähigen Beschichtung versehen ist. Zumindest sollten die Pigmente so aufgebaut sein, daß eine evtl. auftretende Absorption der Beschichtung bzw. der Beschichtungen die Farbtonbrillanz der Interferenzfarbe nicht beeinträchtigt.

Die Lösung dieser Aufgabe gelang durch Belegung von plättchenförmigem Aluminiumpigment mit filmartigen Belägen aus Titanoxiden unterschiedlicher Schichtdicke. Diese mit Titanoxiden belegten Aluminiumpigmente können zusätzlich mit Chrom-III-oxidhydrat (nicht störend im grün-blauen Spektralbereich), oder mit $Fe_2O_3$ (nicht störend im gelb-roten Spektralbereich) belegt sein.

Die erfindungsgemäßen Aluminiumpigmente können nach dem Chemical-Vapor-Depositon-(CVD)-Verfahren hergestellt werden. Hierbei läßt man $TiCl_4$-Dampf in geringer Konzentration mit $H_2O$-Dampf im Wirbelbett in Gegenwart erwärmter bewegter Al-Partikel reagieren. Die stark vereinfachte Bilanzgleichung ist folgende:

$$TiCl_4 + 2H_2O + \text{Al-Pigment} \xrightarrow{N_2,\ T} TiO_2/\text{Al-Pigment} + 4HCl$$

Hiermit gelingt es Interferenzfarben zeigende metallisch glänzende Pigmente herzustellen.

Als Al-Substrat können aus Al-Folie herausgestanzte Al-Plättchen oder nach bekannten Verdüsungs- und Mahltechniken hergestellte Al-Pigmente dienen. Der erwünschte Partikelgrößenbereich liegt zwischen 10 und 120 µm für den mittleren Durchmesser der Plättchen. Die spezifische freie Oberfläche (BET) des Al-Einsatzpigmentes liegt zwischen 0,5 und 5 $m^2/g$.

Die Oberfläche der Plättchen sollte weitgehend frei von Fetten oder anderen Belegungsmitteln sein. Es können handelsübliche Produkte eingesetzt werden.

Im einzelnen wird bei der $TiO_2$-Belegung von plättchenförmigem Al-Pigment so vorgegangen, daß trockenes Al-Pigment in einem beheizbaren Wirbelbettreaktor aus Metall oder Glas eingefüllt und mit inertem Wirbelgas, dem aus Sicherheitsgründen maximal 5 Vol.% Sauerstoff beigemengt sein dürfen, zum Wirbeln gebracht wird. Um den Austrag von feinen Partikeln zu vermeiden, ist der Wirbelreaktor am Kopfende zweckmäßigerweise mit einem Filter versehen.

Das Wirbelgut wird über eine Wandbeheizung oder Strahlungsheizung auf 100 bis 400 °C aufgeheizt. Als Reaktionstemperatur besonders vorteilhaft erwies sich der Temperaturbereich zwischen 180 und 250 °C. Um elektrostatischen Aufladungen vorzubeugen, leitet man in das Wirbelbett während der Aufheizphase Wasserdampf ein. Dies erfolgt zweckmäßigerweise dadurch, daß man das Wirbelgas, oder einen Teil des Wirbelgases, durch temperiertes Wasser leitet, wo es sich mit $H_2O$-Dampf belädt. Wasserdampf kann auch über eine seitlich am Wirbelbettreaktor angebrachten Düse in das Wirbelbett eingetragen werden. Ist die gewünschte Endtemperatur des Wirbelbettes erreicht, so wird über eine weitere, seitlich am Wirbelbett angebrachte, Düse $TiCl_4$-Dampf in das Wirbelbett eingeleitet. Vorteilhaft geht man dabei so vor, daß ein inertes Trägergas, z.B. $N_2$, mit der gewünschten Menge an $TiCl_4$ beladen wird.

Für die Ausbildung qualitativ hochwertiger, d.h. gleichförmiger filmartiger $TiO_2$-Beläge auf der Oberfläche der Al-Partikel ist es wichtig, daß $TiCl_4$ nur in niedriger Konzentration ins Wirbelbett eingetragen wird. Dort kann es mit dem im Überschuß vorhandenen Wasserdampf reagieren.

Versuche haben gezeigt, daß bezogen auf die Gesamtmenge der übrigen ins Wirbelbett eingeleiteten Gase bzw. Dämpfe für $TiCl_4$-Dampf 5 Vol.% nicht überschritten werden dürfen.

Hierbei ist berücksichtigt, daß $H_2O$-Dampf mit stets mehr als 2 Mol/1 Mol $TiCl_4$ vorhanden ist und an der Gesamtmenge der übrigen Gase teilhat.

Mit zunehmender Reaktionsdauer überziehen sich die Al-Plättchen mit einem in seiner Dicke anwachsenden $TiO_2$-Belag. Die $TiO_2$-beschichteten Al-Pigmente zeigen zunächst eine bläuliche, dann gelbe, goldene, rote, violette, grüne, blaue bis gelbe Farbe. Das Beschichtungsverfahren in der Gasphase (Chemial Vapor Deposition) wird zweckmäßigerweise als Chargenverfahren betrieben. Nach Erreichen des gewünschten Interferenzfarbtons wird die $TiCl_4$-Zufuhr abgebrochen, das Wirbelbett wird abgekühlt und ausgetragen. Das während der Reaktion sich bildende HCl verläßt dampfförmig abgasseitig den Reaktor, wo es problemlos entsorgt werden kann.

Die Charakterisierung des nach oben beschriebenem Verfahren hergestellten Interferenzreflexionspigmentes zeigt sehr homogene und äußerst gleichmäßige $TiO_2$-Beläge auf den Al-Substraten. Die Oberfläche der Beschichtung ist glatt. Die Beschichtung selbst besteht aus dichtem polykristallinem $TiO_2$. Eine kristallographische Vorzugsoirientierung ist nicht erkennbar. Die Pigmente weisen einen sehr hohen metallischen Glanz auf. Ihre Farbe ist schichtdickenabhängig.

Der Farbeindruck der beschriebenen Interferenzpigmente läßt sich durch verschiedene zusätzliche Maßnahmen intensivieren. Bekanntlich kann der Farbeindruck von Interferenzfarben verstärkt werden, wenn es gelingt, den Weißsockel des Lichtes abzusenken bzw. zu vermindern. Dies gelingt durch teilweise Reduktion der $TiO_2$-Beschichtung, wobei neben unverändertem $TiO_2$ sich Titanoxide mit einer Oxidationszahl des Titans <4, z.B. dunkles TiO oder gegebenenfalls TiN, bilden. Die partielle Reduktion des $TiO_2$-Belages der $TiO_2$-beschichteten Al-Pigmente kann mit $H_2$, CO, Kohlenwasserstoffen, oder insbesondere Ammoniak, bei Temperaturen von 400 bis 900 °C vorgenommen werden.

Besonders effektiv ist die Reduktion mit Ammoniak bei Temperaturen von 400 bis 660 °C. Die Strömungsgeschwindigkeit des Reduktionsgases sollte dabei 0,5 cm/sec nicht unterschreiten. Außerdem muß das Reduktionsgas trocken sein. Die Reduktion wird zweckmäßigerweise so vorgenommen, daß das zu behandelnde Pigment in einer bewegten Schicht mit dem reduzierenden Gas in Berührung gebracht wird, z.B. in einem Drehrohr oder einer Drehtrommel mit Stolperstufen oder in einem Wirbelschichtreaktor. Die Reduktionszeit beträgt 30 bis 360 Min. Mit zunehmender Reduktionszeit wird das behandelte Einsatzprodukt zunehmend dunkler, was auf einen steigenden Anteil an TiO oder TiN oder Titanoxinitriden zurückzuführen ist.

Tatsächlich gelingt es, durch das Dunklerstellen der eingesetzten Interferenzreflexionspigmente die jeweilige Interferenzfarbe des Einsatzproduktes verstärkt in Erscheinung treten zu lassen. Beispielsweise erscheint ein $TiO_2$-beschichtetes Al-Pigment mit einer schwach blauen Interferenzfarbe nach der reduzierenden Behandlung mit $NH_3$ bei 600 °C über 1 Stunde intensiv blau.

Die Farbe der mit Titanoxiden beschichteten Aluminiumpigmente läßt sich in einigen Spektralbereichen außerdem durch weitere anorganische Beschichtungen intensivieren. So ist es möglich, rote Pigmente durch eine anteilige (in Bezug auf die Schichtdicke) Eisenrot-($Fe_2O_3$)-Belegung farbintensiver zu machen. Es wurde auch gefunden, daß grüne Pigmente durch eine anteilige Chromoxid- oder CrOOH-Beschichtung intensiver grün hergestellt werden können. Solche stofflich zusätzlichen anorganischen Beschichtungen haben den weiteren Vorteil, daß die wegen seiner Photoaktivität im Außenbereich nur bedingt verwendbare $TiO_2$-Beschichtung stabilisiert wird. Insofern sind auch zusätzliche Beschichtungen des $TiO_2$/Al-Pigmentes mit weiteren nichtfarbigen Oxiden wie $SiO_2$, $Al_2O_3$ oder $ZrO_2$ vorteilhaft.

Zusätzliche Beschichtungen können nach bekannten Methoden im wäßrigen Medium durch Hydrolyse der entsprechenden Salzlösungen mit anschließendem Waschen und Trocknen der Pigmente aufgebracht werden. Eleganter werden weitere Beschichtungen aus der Gasphase aufgebracht, da sie im direkten Anschluß an die $TiO_2$-Beschichtung z.B. in einem Wirbelbett erfolgen können. Mit den leicht verdampfbaren Chloriden des Siliciums und Aluminiums erfolgt die $SiO_2$- und $Al_2O_3$- oder eine wechselweise $SiO_2/Al_2O_3$-Belegung in direkter Analogie zur $TiO_2$-Belegung.

Auch eine zusätzliche Eisenoxidbelegung läßt sich vorteilhaft über CVD-Methoden durchführen. Hierbei wird im gleichen Wirbelbett-Reaktor, ohne abzukühlen, anstelle des $TiCl_4$-Dampfes Eisenpentacarbonyldampf in den Reaktor eingedüst. Die Konzentration des Eisenpentacarbonyls darf dabei, gerechnet auf das Gesamtvolumen der übrigen in das Wirbelbett geleiteten Gase 5 Vol.% nicht überschreiten. $Fe(CO)_5$-Dampf reagiert im Wirbelbett mit dem über das Wirbelgas eingeleiteten Sauerstoff bei Temperaturen oberhalb von 150 °C, vorzugsweise bei Temperaturen von 180 bis 250 °C, entsprechend der folgenden Bilanzgleichung:

$$2Fe(CO)_5 + \tfrac{13}{2} O_2 + TiO_2/\text{Al-Pigment} \xrightarrow{N_2,\,T} Fe_2O_3/TiO_2/\text{Al-Pigment} + 5CO_2$$

Über die Dauer der Reaktionszeit läßt sich die Dicke der zusätzlichen $Fe_2O_3$-Belegung steuern. Auch alternierendes Beschichten mit $TiO_2$ und $Fe_2O_3$ ist möglich.

Grundsätzlich ist zu sagen, daß bei zusätzlichen Beschichtungen mit den genannten Metalloxiden die Gesamtdicke der vorgesehenen Beschichtung nicht geändert werden soll, wenn nicht eine Änderung der Interferenzfarbe in Kauf genommen werden soll.

Röntgenographisch läßt sich in der Beschichtung alternierend belegter Pigment $TiO_2$ und $Fe_2O_3$ als separate Phase nachweisen. Bewitterungsversuche zeigten, daß die mit $Fe_2O_3$-beschichteten Titanoxid-Aluminiumpigmente hervorragende Wetterechtheit aufweisen, so daß ihre Verwendbarkeit im Außenbereich z.B. für die Herstellung von Automobillackierungen gewährleistet ist.

Die erfindungsgemäßen Reflexionspigmente können neben der Einfärbung von Lacken aber auch zum Einfärben von Kunststoffen, Druckfarben, keramischen Artikeln, Gläsern und von kosmetischen Produkten verwendet werden.

Die folgenden Versuche erläutern beispielhaft die Erfindung:

In den nachfolgend aufgeführten Beispielen 1 bis 4 wurde folgende Apparatur benutzt:

Mit Infrarot-Strahlern beheizbarer Wirbelbettreaktor aus Glas mit konischem Wirbelgaseintritt an der Unterseite und mit durch Stickstoffpulse abreinigbaren Filterstrümpfen an der Oberseite, Durchmesser 60 mm, Höhe 1000 mm, zwei seitlich auf ein Drittel Höhe angebrachte Düsen.

Alle angegebenen Gasmengen sind bei 20 °C und bei 1,013 bar gemessen.

Beispiel 1

In den Wirbelbettreaktor werden 300 g eines handelsüblichen Aluminiumpigmentes mit einer BET-Oberfläche von 1,5 $m^2/g$ und einem mittleren Teilchendurchmesser von 60 $\mu m$ (90 % der Teilchen liegen zwischen 35 und 90 $\mu m$) eingefüllt. Durch Einblasen von 600 l/h Stickstoff und 100 l/h Luft an der unteren Öffnung des Konus wird das Pigment fluidisiert. Der Luftstrom wird durch auf 50 °C geheiztes Wasser geleitet. Mit Hilfe der IR-Strahler wird die Wirbelbettinnentemperatur auf Werte zwischen 192 und 228 °C gebracht. Nach Erreichen dieser Temperatur wird ein Stickstoffstrom von 300 l/h, der durch Einleiten in einen mit $TiCl_4$ gefüllten, auf 50 °C temperierten Sättigerkolben mit Titantetrachlorid-Dampf beladen ist, durch eine Düse in den Ofen geblasen. Das Titantetrachlorid reagiert mit dem mit dem Luftstrom eingebrachten Wasserdampf zu Titandioxid und Chlorwasserstoff. Unter den gewählten Reaktionsbedingungen scheidet sich das gebildete Titandioxid spontan als Film auf den Aluminiumplättchen ab. Insgesamt

werden über einen Zeitraum von 12 Stunden 400 ml $TiCl_4$ in das Wirbelbett eingetragen, wobei nach 50, 100, 150, 170, 190, 210, 230, 250, 270, 290, 310 und 350 ml $TiCl_4$ jeweils eine kleine Pigmentprobe entnommen wird. Ausbeute: 460 g $TiO_2$-beschichtetes Aluminiumpigment mit 28,0 Gew.% Titan.

Zur Beurteilung der Koloristik der erfindungsgemäß hergestellten Pigmente werden je 0,4 g der Pigmentproben in 3,6 g eines Polyester-Mischlackes mit 21 Gew.% Feststoffanteil eingerührt und 2 Minuten lang im Red Devil dispergiert. Mit einem Spiralrakel (80 µm Naßfilmdicke) werden auf einem schwarzweißen Karton Rakelabzüge der pigmentierten Lacke angefertigt. Die Messung der CIELAB-Farbwerte erfolgt mit einem DATACOLOR Spektralphotometer MCS 111 mit Metallic-Meßkopf GK 111 bei einer Winkel-Differenz von 20° zum Glanzwinkel. Die Angaben der Farbwerte (L*, a* und b*) beziehen sich auf die Normlichtart D 65. Dabei entspricht L* der Helligkeit, a* dem Rot- bzw. Grünanteil und b* dem Blau- bzw. Gelbanteil. Alle Lackierungen zeigen den von Aluminiumpigmenten bekannten hohen metallischen Glanz. Zusätzlich weisen sie in Abhängigkeit von der auf das Aluminiumpigment aufgebrachten Titandioxidmenge pastellartige Interferenzfarben in der Reihenfolge blau, gold, rot, violett und grün auf. Ab einem Titangehalt von 20 Gew.% werden Interferenzfarben höherer Ordnung erhalten.

Beispiel 2

In den Wirbelbettreaktor werden 200 g eines handelsüblichen Aluminiumpigmentes mit einer BET-Oberfläche von 1,5 $m^2/g$ und einem mittleren Teilchendurchmesser von 60 µm (90 % der Teilchen liegen zwischen 35 und 90 µm) eingefüllt. Durch Einblasen von 400 l/h Stickstoff, der durch Einleiten in 50°C warmes Wasser mit Wasserdampf angereichert ist, an der unteren Öffnung des Konus wird das Pigment fluidisiert. Mit Hilfe der IR-Strahler wird die Wirbelbettinnentemperatur auf Werte zwischen 210 und 220°C gebracht. Nach Erreichen dieser Temperatur wird ein Stickstoffstrom von 150 l/h, der durch Einleiten in einen mit $TiCl_4$ gefüllten, auf 50°C temperierten Sättigerkolben mit Titantetrachlorid-Dampf beladen wird, durch eine Düse in den Ofen geblasen. Das Titantetrachlorid reagiert mit dem mit dem Stickstoffstrom eingebrachten Wasserdampf zu Titandioxid und Chlorwasserstoff. Unter den gewählten Reaktionsbedingungen scheidet sich das gebildete Titandioxid spontan als Film auf den Aluminiumplättchen ab. Insgesamt werden über einen Zeitraum von 2,5 Stunden 20 ml $TiCl_4$ in das Wirbelbett eingetragen. Ein Rakelabzug einer Pigmentprobe - nach Beispiel 1 angefertigt - zeigt hohen metallischen Glanz mit blauem Schimmer.

Beispiel 3

In den Wirbelbettreaktor wird eine Mischung aus 150 g handelsüblichem Aluminiumpigment mit einer BET-Oberfläche von 1,5 $m^2/g$ und einem mittleren Teilchendurchmesser von 60 µm (90 % der Teilchen liegen zwischen 35 und 90 µm) und 150 g handelsüblichem Aluminiumpigment mit einer BET-Oberfläche von 4,5 $m^2/g$ und einem mittleren Teilchendurchmesser von 20 µm (90 % der Teilchen liegen zwischen 6 und 35 µm) eingefüllt. Durch Einblasen von 150 l/h Stickstoff und 180 l/h Luft an der unteren Öffnung des Konus wird das Pigment fluidisiert und homogenisiert. Der Luftstrom wird durch auf 50°C geheiztes Wasser geleitet. Mit Hilfe der IR-Strahler wird die Wirbelbettinnentemperatur auf Werte zwischen 195 und 200°C gebracht. Nach Erreichen dieser Temperatur wird mit Hilfe eines Stickstoffstromes von 200 l/h, der vorher durch auf 50°C geheiztes Wasser geleitet wird, Wasserdampf in den Reaktor eingeführt und der Wirbelgasstrom durch Einleiten in einen mit $TiCl_4$ gefüllten, auf 50°C temperierten Sättigerkolben mit Titantetrachlorid-Dampf beladen. Unter den gewählten Reaktionsbedingungen scheidet sich das gebildete Titandioxid spontan als Film auf den Aluminiumplättchen ab. Insgesamt werden über einen Zeitraum von 12 Stunden 270 ml $TiCl_4$ in das Wirbelbett eingetragen. Das Pigment zeigt metallischen Glanz mit rötlichem Schimmer. Ein Rakelabzug dieses Produktes zeigt die CIELAB-Farbwerte L* = 107,1; a* = 2,2; b* = 7,6.

Im Anschluß an die $TiO_2$-Beschichtung wird ein Stickstoffstrom von 200 l/h, der durch Einleiten in einen mit $Fe(CO)_5$ gefüllten, auf 50°C temperierten Sättigerkolben mit Eisenpentacarbonyl-Dampf beladen wird, durch eine Düse in den Ofen geblasen. Das Eisencarbonyl reagiert mit dem dort vorhandenen Sauerstoff zu Eisenoxid (HZämatit) und Kohlendioxid. Unter den gewählten Reaktionsbedingungen scheidet sich das gebildete $Fe_2O_3$ spontan als Film auf den $TiO_2$-beschichteten Aluminiumplättchen ab. Insgesamt werden über einen Zeitraum von 0,5 Stunden 10 ml ($Fe(CO)_5$ in das Wirbelbett eingetragen. Ein Rakelabzug einer Pigmentprobe zeigt hohen metallischen Glanz mit intensiv rotem Schimmer mit den CIELAB-Farbwerten L* = 102,8; a* = 5,8; b* = 8,9.

Beispiel 4

In den Wirbelbettreaktor wird eine Mischung aus 150 g handelsüblichem Aluminiumpigment mit einer BET-Oberfläche von 1,5 $m^2/g$ und einem mittleren Teilchendurchmesser von 60 $\mu m$ (90 % der Teilchen liegen zwischen 35 und 90 $\mu m$) und 150 g handelsüblichem Aluminiumpigment mit einer BET-Oberfläche von 4,5 $m^2/g$ und einem mittleren Teilchendurchmesser von 20 $\mu m$ (90 % der Teilchen liegen zwischen 6 und 35 $\mu m$) eingefüllt. Durch Einblasen von 150 l/h Stickstoff und 180 l/h Luft an der unteren Öffnung des Konus wird das Pigment fluidisiert und homogenisiert. Der Luftstrom wird durch auf 50 °C geheiztes Wasser geleitet. Mit Hilfe der IR-Strahler wird die Wirbelbettinnentemperatur auf Werte zwischen 195 und 200 °C gebracht. Nach Erreichen dieser Temperatur wird mit Hilfe eines Stickstoffstromes von 200 l/h, der vorher durch auf 50 °C geheiztes Wasser geleitet wird, Wasserdampf in den Reaktor eingeführt und der Wirbelgasstrom durch Einleiten in einen mit $TiCl_4$ gefüllten, auf 50 °C temperierten Sättigerkolben mit Titantetrachlorid-Dampf beladen. Unter den gewählten Reaktionsbedingungen scheidet sich das gebildete Titandioxid spontan als Film auf den Aluminiumplättchen ab. Insgesamt werden über einen Zeitraum von 7 Stunden 130 ml $TiCl_4$ in das Wirbelbett eingetragen. Das Pigment weist eine dunkelblaue Farbe auf. Ein Rakelabzug einer Pigmentprobe - nach Beispiel 1 angefertigt - zeigt hohen metallischen Glanz mit intensiv blauem Schimmer.

Beispiel 5

25 g des nach Beispiel 4 hergestellten blauen Interferenzpigmentes werden in einen beheizbaren 250 ml Quarzdrehkolben mit eingebauten, 0,5 cm breiten Stolperleisten eingetragen und unter Stickstoffatmosphäre unter Drehen des Kolbens auf 600 °C aufgeheizt. Dann wird getrocknetes $NH_3$-Gas mit einem Durchsatz von 30 l/h 60 Min. lang über das $TiO_2$-beschichtete Al-Pigment geleitet. Danach wird unter einem Stickstoffstrom 3 Stunden lang abgekühlt.

Das Produkt ist intensiv blau mit einem leichten Rotstich. Mikroskopische Aufnahmen zeigen, daß das Pigment seine Plättchenform beibehalten hat. Röntgenaufnahmen lassen TiN bzw. TiO (nicht unterscheidbar) erkennen. Die Analyse ergibt einen Gehalt von 1 Gew.% $N^{3-}$.

## Patentansprüche

**Patentansprüche für folgende Vertragsstaaten : BE, CH, DE, FR, GB, IT, LI, NL, SE**

1. Metallisch glänzende Reflexionspigmente aus einem Substrat aus plättchenförmigem Aluminium und einer Beschichtung von Titanoxiden, ausgenommen solche Pigmente, bei denen die Titanoxidschicht durch wäßrige Hydrolyse von Titantetraisopropanolat in Isopropanol abgeschieden wurde.

2. Reflexionspigmente nach Anspruch 1, dadurch gekennzeichnet, daß die Beschichtung aus Titandioxid besteht.

3. Reflexionspigmente nach Anspruch 1, dadurch gekennzeichnet, daß die Beschichtungen Oxide des Titans mit einer Oxidationszahl des Titans $<4$ enthalten.

4. Reflexionspigmente nach Anspruch 3, dadurch gekennzeichnet, daß sie Titannitrid und/oder Titanoxinitrid enthalten.

5. Reflexionspigmente nach Ansprüchen 1 bis 4, dadurch gekennzeichnet, daß sie zusätzlich Beschichtungen aus anderen Metalloxiden aufweisen.

6. Reflexionspigmente nach Anspruch 5, dadurch gekennzeichnet, daß die anderen Metalloxide Eisenoxid sind.

7. Verfahren zur Herstellung der Reflexionspigmente nach Ansprüchen 1 und 2, dadurch gekennzeichnet, daß man $TiCl_4$-Dampf in eine mit einem inerten Trägergas aufrechterhaltene Wirbelschicht aus plättchenförmigem Aluminium, bei Temperaturen oberhalb von 100 °C, zusammen mit Wasserdampf einleitet, mit der Maßgabe, daß das Volumen der $TiCl_4$-Dämpfe, bezogen auf das Volumen der anderen in die Wirbelschicht eingeleiteten Gase und Dämpfe 5 Vol.% nicht überschreitet.

**8.** Verfahren zur Herstellung von Reflexionspigmenten nach Ansprüchen 1, 3 und 4, dadurch gekennzeichnet, daß man Reflexionspigmente gemäß Anspruch 2, einer Behandlung mit reduzierenden Gasen, insbesondere mit Ammoniak, bei Temperaturen von 400 bis 900 °C unterwirft.

**9.** Verfahren zur Herstellung von Reflexionspigmenten nach Ansprüchen 5 bis 6, dadurch gekennzeichnet, daß man dampfförmiges Eisenpentacarbonyl in eine mit einem inerten Trägergas aufrechterhaltene Wirbelschicht aus mit Titanoxiden belegten Reflexionspigmenten einleitet und bei Temperaturen oberhalb von 150 °C oxidiert, mit der Maßgabe, daß die Menge des in die Wirbelschicht eingeführten Eisenpentacarbonyls, bezogen auf die insgesamt in die Wirbelschicht eingeführten Gase, 5 Vol.% nicht überschreitet.

**10.** Verwendung der Reflexionspigmente gemäß Ansprüchen 1 bis 6 zum Einfärben von Lacken, Kunststoffen, Druckfarben, keramischen Artikeln, Gläsern und von kosmetischen Produkten.

**Patentansprüche für folgenden Vertragsstaat : ES**

**1.** Verfahren zur Herstellung von Reflexionspigmenten aus einem Substrat aus plättchenförmigem Aluminium und einer Beschichtung aus Titanoxiden, dadurch gekennzeichnet, daß man $TiCl_4$-Dampf in eine mit einem inerten Trägergas aufrechterhaltene Wirbelschicht aus plättchenförmigem Aluminium, bei Temperaturen oberhalb von 100 °C, zusammen mit Wasserdampf einleitet, mit der Maßgabe, daß das Volumen der $TiCl_4$-Dämpfe, bezogen auf das Volumen der anderen in die Wirbelschicht eingeleiteten Gase und Dämpfe 5 Vol.% nicht überschreitet.

**2.** Verfahren zur Herstellung von Reflexionspigmenten nach Anspruch 1, dadurch gekennzeichnet, daß man die Reflexionspigmente anschließend einer Behandlung mit reduzierenden Gasen, insbesondere mit Ammoniak, bei Temperaturen von 400 bis 900 °C unterwirft.

**3.** Verfahren zur Herstellung von Reflexionspigmenten nach Ansprüchen 1 bis 2, dadurch gekennzeichnet, daß man dampfförmiges Eisenpentacarbonyl in eine mit einem inerten Trägergas aufrechterhaltene Wirbelschicht aus mit Titanoxiden belegten Reflexionspigmenten einleitet und bei Temperaturen oberhalb von 150 °C oxidiert, mit der Maßgabe, daß die Menge des in die Wirbelschicht eingeführten Eisenpentacarbonyls, bezogen auf die insgesamt in die Wirbelschicht eingeführten Gase, 5 Vol.% nicht überschreitet.

**Claims**

**Claims for the following Contracting States : BE, CH, DE, FR, GB, IT, LI, NL, SE**

**1.** Metallically bright reflection pigments comprising a substrate of plateletlike aluminum and a coating of titanium oxides, excepting those pigments where the titanium oxide layer was deposited by aqueous hydrolysis of titanium tetraisopropoxide in isopropanol.

**2.** Reflection pigments as claimed in claim 1, wherein the coating consists of titanium dioxide.

**3.** Reflection pigments as claimed in claim 1, wherein the coatings contain oxides of titanium where the titanium is in an oxidation state <4.

**4.** Reflection pigments as claimed in claim 3, containing titanium nitride and/or titanium oxynitride.

**5.** Reflection pigments as claimed in any of claims 1 to 4, additionally including coatings comprising other metal oxides.

**6.** Reflection pigments as claimed in claim 5, wherein the other metal oxides are iron oxide.

**7.** A process for preparing the reflection pigments of claims 1 and 2, which comprises passing $TiCl_4$ vapor at above 100 °C together with water vapor into a fluidized bed of plateletlike aluminum suspended in an inert carrier gas, with the proviso that the volume of the $TiCl_4$ vapors, based on the volume of the other gases and vapors introduced into the fluidized bed, does not exceed 5 % by volume.

8. A process for preparing reflection pigments of claims 1, 3 and 4, which comprises subjecting reflection pigments of claim 2 to treatment with reducing gases, particularly with ammonia, at from 400 to 900 °C.

9. A process for preparing reflection pigments as claimed in claims 5 and 6, which comprises passing vaporous iron pentacarbonyl into a fluidized bed of titanium oxide-coated reflection pigments suspended in an inert carrier gas and oxidizing at above 150 °C, with the proviso that the amount of iron pentacarbonyl introduced into the fluidized bed does not exceed 5% by volume, based on the total of gases introduced into the fluidized bed.

10. The use of the reflection pigments as claims 1 to 6 for coloring paints, plastics printing inks, ceramic articles, glasses and cosmetic products.

**Claims for the following Contracting State : ES**

1. A process for preparing reflection pigments comprising a substrate of plateletlike aluminum and a coating of titanium oxides, which comprises passing $TiCl_4$ vapor at above 100 °C together with water vapor into a fluidized bed of plateletlike aluminum suspended in an inert carrier gas, with the proviso that the volume of the $TiCl_4$ vapors, based on the volume of the other gases and vapors introduced into the fluidized bed, does not exceed 5 % by volume.

2. A process for preparing reflection pigments as claimed in claim 1, wherein the reflection pigments are subsequently subjected to a treatment with reducing gases, particularly with ammonia, at from 400 to 900 °C.

3. A process for preparing reflection pigments as claimed in claims 1 and 2, wherein vaporous iron pentacarbonyl is passed into a fluidized bed of titanium oxide-coated reflection pigments suspended in an inert carrier gas and oxidized at above 150 °C, with the proviso that the amount of iron pentacarbonyl introduced into the fluidized bed does not exceed 5% by volume, based on the total of gases introduced into the fluidized bed.

**Revendications**
**Revendications pour les Etats contractants suivants : BE, CH, DE, FR, GB, IT, LI, NL, SE**

1. Pigments à réflexion à éclat métallique, constitués d'un support d'alumium en forme de plaquettes et d'un revêtement d'oxydes de titane, à l'exclusion des pigments du genre de ceux dans lesquels la couche d'oxyde de titane a été déposée par hydrolyse aqueuse de tétraisopropylate dans l'isopropanol.

2. Pigments à réflexion selon la revendication 1, caractérisés en ce que le revêtement est constitué de dioxyde de titane.

3. Pigments à réflexion selon la revendication 1, caractérisés en ce que les revêtements d'oxydes du titane contiennent un indice d'oxydation du titane inférieur à 4.

4. Pigments à réflexion suivant la revendication 3, caractérisés en ce qu'ils contiennent du nitrure de titane et/ou de l'oxynitrure de titane.

5. Pigments à réflexion suivant l'une quelconque des revendications 1 à 4, caractérisés en ce qu'ils comportent complémentairement des revêtements constitués d'autres oxydes de métaux.

6. Pigments à réflexion suivant la revendication 5, caractérisés en ce que les autres oxydes de métaux sont constitués d'oxyde de fer.

7. Procédé de préparation des pigments à réflexion selon les revendications 1 et 2, caractérisé en ce que l'on introduit de la vapeur de $TiCl_4$ dans une couche fluidisée d'aluminium en forme de plaquettes, maintenue en mouvement tourbillonnant par un gaz de support inerte, à des températures supérieures à 100 °C, en même temps que de la vapeur d'eau, avec la condition que le volume des vapeurs de $TiCl_4$, par rapport au volume des autres vapeurs et gaz introduits dans la couche fluidisée ne dépasse pas 5% volumiques.

8. Procédé de préparation de pigments à réflexion selon les revendications 1, 3 et 4, caractérisé en ce que l'on soumet des pigments à réflexion selon la revendication 2 à un traitement par des gaz réducteurs, plus particulièrement l'ammoniac, à des températures de 400 à 900 °C.

9. Procédé de préparation de pigments à réflexion selon les revendications 5 et 6, caractérisé en ce que l'on introduit du fer pentacarbonyle sous forme de vapeur dans une couche fluidisée de pigments à réflexion porteurs d'oxydes du titane, maintenue en mouvement turbulent par un gaz de support inerte et on l'oxyde à des températures supérieures à 150 °C, avec la condition que la quantité du fer pentacarbonyle introduite dans la couche fluidisée, par rapport aux gaz introduits au total dans la couche fluidisée, ne dépasse pas 5% volumiques.

10. Utilisation des pigments à réflexion selon les revendications 1 à 6 pour la coloration de laques ou peintures, de matières plastiques, d'encres d'impressions, d'articles en matières céramiques, de verres et de produits cosmétiques.

**Revendications pour l'Etat contractant suivant : ES**

1. Procédé de préparation de pigments à réflexion constitués d'un support formé d'aluminium sous forme de plaquettes et d'un revêtement d'oxydes du titane, caractérisé en ce que l'on introduit de la vapeur de $TiCl_4$ dans une couche fluidisée d'aluminium en forme de plaquettes, maintenue en mouvement tourbillonnant par un gaz de suppoprt inerte, à des températures supérieures à 100 °C, en même temps que de la vapeur d'eau, avec la condition que le volume des vapeurs de $TiCl_4$, par rapport au volume des autres vapeurs et gaz introduits dans la couche fluidisée ne dépasse pas 5% volumiques.

2. Procédé de préparation de pigments à réflexion selon la revendication 1, caractérisé en ce que l'on soumet des pigments à réflaxion selon la revendication 1 à un traitement par des gaz réducteurs, plus particulièrement l'ammoniac, à des températures de 400 à 900 °C.

3. Procédé de préparation de pigments à réflexion selon les revendications 1 et 2, caractérisé en ce que l'on introduit du fer pentacarbonyle sous forme de vapeur dans une couche fluidisée de pigments à réfléxion porteurs d'oxyde du titane, maintenue en mouvemant turbulent par un gaz de support inerte et on l'oxyde à des températures supérieures à 150 °C, avec la condition que la quantité de fer pentacarbonyle introduit dans la couche fluidisée, par rapport aux gaz introduits au total dans la couche fluidisée, ne dépasse pas 5% volumiques.